Europäisches Patentamt

European Patent Office

Office européen des brevets

(19)

(11) Veröffentlichungsnummer: **0 333 047**

**A2**

(12) **EUROPÄISCHE PATENTANMELDUNG**

(21) Anmeldenummer: 89104229.3

(22) Anmeldetag: 09.03.89

(51) Int. Cl.4: **C07C 128/00**

(30) Priorität: 16.03.88 DE 3808766

(43) Veröffentlichungstag der Anmeldung:
20.09.89 Patentblatt 89/38

(84) Benannte Vertragsstaaten:
AT BE CH DE ES FR GB IT LI NL SE

(71) Anmelder: SKW Trostberg Aktiengesellschaft
Dr.-Albert-Frank-Strasse 32 Postfach 12 62
D-8223 Trostberg(DE)

(72) Erfinder: Grambow, Clemens Dr.
Haushofer Strasse 26 a
D-8221 Seebruck(DE)
Erfinder: Beck, Ferdinand, Dr.
Angerweg 2
D-8223 Trostberg(DE)

(74) Vertreter: Huber, Bernhard, Dipl.-Chem. et al
Möhlstrasse 22 Postfach 860 820
D-8000 München 86(DE)

(54) Verfahren zur Gewinnung von Guanidinhydrohalogeniden aus den bei der Herstellung von Mercaptoalkylsilanen anfallenden Nebenproduktgemischen.

(57) Es wird ein Verfahren zur Rückgewinnung von Guanidinhydrohalogeniden aus den bei der Herstellung von Mercaptoalkylsilanen anfallenden Produktgemischen beschrieben, welche im wesentlichen aus Guanidinhydrohalogenid, Ammoniumhalogenid, Thioharnstoff und Silanverbindungen bestehen, wobei man die organischen Verunreinigungen aus dem Produktgemisch mit einem aliphatischen Keton mit bis zu sieben Kohlenstoffatomen extrahiert und den Extrakt vom Rückstand beispielsweise durch Filtration abtrennt. Auf dieses Weise läßt sich das Guanidinhydrohalogenid in sehr hoher Reinheit zurückgewinnen. Außerdem fallen die organischen Verunreinigungen in einer solchen Form an, die eine problemlose Verwertung bzw. Beseitigung ermöglichen.

EP 0 333 047 A2

## Verfahren zur Gewinnung von Guanidinhydrohalogeniden aus den bei der Herstellung von Mercaptoalkylsilanen anfallenden Nebenproduktgemischen

Die Erfindung betrifft ein Verfahren zur Gewinnung von Guanidinhydrohalogenid aus den bei der Herstellung von Mercaptoalkylsilanen anfallenden Produktgemischen.

Aus der DE-PS 33 46 910 ist ein Verfahren zur Herstellung von Mercaptoalkylsilanen bekannt, gemäß dem Halogenalkylsilane mit Thioharnstoff und Ammoniak umgesetzt werden. Das dabei als Nebenprodukt anfallende Guanidinhydrohalogenid, vorzugsweise Guanidinhydrochlorid, wird durch Behandlung der Reaktionslösung mit Chlorkohlenwasserstoffen ausgefällt und anschließend beispielsweise durch Filtration abgetrennt.

Das auf diese Weise abgetrennte Nebenprodukt enthält durchschnittlich nur ca. 65 bis 75 Gew.-% Guanidinhydrochlorid und ist verunreinigt mit ca. 5 bis 10 Gew.-% Ammoniumchlorid, 5 bis 10 Gew.-% Thioharnstoff, 10 bis 15 Gew.-% Silanverbindungen sowie 2 bis 5 Gew.-% flüchtigen Verbindungen wie z.B. Chlorkohlenwasserstoffen und übelriechenden Mercaptanen.

Durch den relativ hohen Gehalt an Verunreinigungen ist dieses Nebenprodukt für die üblichen Einsatzgebiete von Guanidinsalzen beispielsweise als Synthesebaustein, insbesondere für pharmazeutische Produkte völlig ungeeignet. Eine Deponierung kann wegen der guten Wasserlöslichkeit und der ökotoxikologischen Bedenklichkeit einiger Bestandteile sowie wegen des Geruchs nur unter einschneidenden Sicherheitsvorkehrungen erfolgen.

Eine Verbrennung dieses Nebenproduktgemisches erfordert wegen der chemischen Reaktionsträgheit einzelner Komponenten sowie des hohen Stickstoff- und Chlorgehaltes einen sehr hohen Aufwand an zusätzlicher Energie. Darüber hinaus entstehen bei der Verbrennung zwangsläufig große Mengen an sauren Abgasen wie HCl, $SO_2$ und $NO_x$.

Der vorliegenden Erfindung liegt daher die Aufgabe zugrunde, ein Verfahren zur Gewinnung von Guanidinhydrohalogeniden aus den bei der Herstellung von Mercaptoalkylsilanen anfallenden Nebenproduktgemischen zu entwickeln, welche im wesentlichen aus Guanidinhydrohalogenid, Ammoniumhalogenid, Thioharnstoff und Silanverbindungen bestehen.

Diese Aufgabe wurde erfindungsgemäß dadurch gelöst, daß man die organischen Verunreinigungen aus dem Produktgemisch mit einem aliphatischen Keton mit bis zu sieben Kohlenstoffatomen extrahiert und den Extrakt vom Rückstand beispielsweise durch Filtration abtrennt.

Es hat sich nämlich überraschenderweise gezeigt, daß man auf diese Weise das Guanidinhydrohalogenid in sehr hoher Reinheit gewinnen kann.

Außerdem fallen die vom Guanidinhydrochlorid abgetrennten organischen Verunreinigungen in einer Form an, die eine problemlose Verwertung bzw. Beseitigung ermöglicht, was ebenfalls nicht vorhersehbar war.

Beim Verfahren der Erfindung wird das Nebenproduktgemisch, welches bei der Herstellung von Mercaptoalkylsilanen beispielsweise gemäß der DE-PS 33 46 910 anfällt und im Mittel aus ca. 65 bis 75 Gew.-% Guanidinhydrohalogenid (Bromid- oder Chlorid), 5 bis 10 Gew.-% Ammoniumbromid oder -chlorid, 5 bis 10 Gew.-% Thioharnstoff, 10 bis 15 Gew.-%Silanverbindungen sowie 2 bis 5 Gew.-% flüchtige Verbindungen besteht, einer Extraktion mit einem aliphatischen Keton mit bis zu sieben Kohlenstoffatomen unterworfen.

Als Ketone im Rahmen der vorliegenden Erfindung werden symmetrische oder unsymmetrische Ketone mit aliphatischen Resten, welche verzweigt oder ungesättigt sein können, wie z.B. Methyl, Äthyl, Vinyl, Propyl, Isopropyl eingesetzt.

Vorzugsweise werden Ketone mit drei bis vier Kohlenstoffatomen und unverzweigten Resten wie z.B. Methyläthylketon, Methylvinylketon und insbesondere Aceton verwendet.

Die Menge des für die Extraktion eingesetzten Ketons richtet sich im wesentlichen nach dem Gewicht des Nebenproduktgemisches und beträgt vorzugsweise die ein-bis zweifache Menge. Die Extraktion erfolgt hierbei zweckmäßig durch Digerieren des im Keton aufgeschlämmten Nebenproduktgemisches. Man kann auch größere Mengen an Keton einsetzen, doch werden diese zusätzlichen Mengen sehr schnell unwirtschaftlich, weil die anschließende Entfernung des Lösungsmittels hierdurch nur unnötig große Energiekosten verursacht.

Das in dem entsprechenden Keton aufgeschlämmte Produktgemisch wird zweckmäßigerweise mit den technisch üblichen Vorrichtungen durchmischt. Bei der Extraktion lösen sich die organischen Verunreinigungen wie z.B. Thioharnstoff praktisch selektiv im Keton, während das Guanidinhydrohalogenid und das Ammoniumhalogenid als unlöslicher Rückstand zurückbleiben.

Die Extraktion kann sowohl bei Raumtemperatur als auch bei erhöhter Temperatur erfolgen, die jedoch unterhalb des Siedepunktes des eingesetzten Ketons liegen sollte. Vorzugsweise wird jedoch bei Raumtemperatur gearbeitet.

Die Extraktion kann in einer oder mehreren Stufen erfolgen, wobei die Mehrstufenextraktion auch als Gegenstromextraktion technisch sinnvoll durchgeführt werden kann.

In einer bevorzugten Ausführungsform wird die Extraktion in zwei Stufen durchgeführt, wobei die Lösung nach der ersten Extraktionsstufe (Filtrat I) in üblicher Weise vom Feststoff beispielsweise durch Filtration abgetrennt wird. Anschließend wird der Extraktionsrückstand in der zweiten Extraktionsstufe mit frischem Lösungsmittel extrahiert und das dabei entstehende Filtrat II wieder für die 1. Extraktionsstufe der nächsten Charge als Lösungsmittel eingesetzt.

In Abhängigkeit von den wirtschaftlichen Gegebenheiten können zur Erzielung besonders hoher Reinheiten des Guanidinhydrohalogenids beliebig viele weitere Extraktionsstufen in analoger Weise durchgeführt werden.

Durch dieses technisch sehr einfache Verfahren lassen sich schon in zwei Extraktionsstufen mehr als 95 % der organischen Verunreinigungen entfernen und man gewinnt ein Guanidinhydrohalogenid mit einer Reinheit von 90 bis 95 %, welches noch ca. 5 bis 9 Gew.-% Ammoniumsalz sowie ca. 0,3 bis 0,8 Gew.-% Verunreinigungen (Thioharnstoff, Silane, Mercaptane) enthält.

Die beim erfindungsgemäßen Verfahren anfallenden Ketonextrakte, welche die organischen Verunreinigungen enthalten, können durch Einengen und Rückgewinnen des Ketons ebenfalls weiterverarbeitet werden.

Nach dem Abziehen des Lösungsmittels entsteht ein Feststoff mit 35 bis 45 Gew.-% Thioharnstoff sowie 40 bis 60 Gew.-% Silanen, der auf verschiedene Weise problemlos entsorgt werden kann.

Der nach dem erfindungsgemäßen Verfahren aus dem Filtrat gewonnene Rückstand läßt sich vergleichsweise günstig deponieren. Auf diese Weise werden ca. 75 bis 85 % des ursprünglich für das Nebenprodukt benötigten Deponierraumes eingespart. Außerdem ist die Deponie der verbleibenden Stoffe mit geringeren Risiken verbunden, da die leicht wasserlöslichen und korrosiven Guanidinsalze weitgehend entfernt sind.

Als weitere Entsorgungsmöglichkeit dieses Rückstandes bietet sich die Verbrennung an, wobei die Menge hierbei um 75 bis 85 % reduziert wird. Durch die starke Reduzierung der stickstoff- und chlorhaltigen Substanzen zeigt diese Restmenge darüber hinaus günstigere Werte beim spezifischen Energieverbrauch (besserer Brennwert) und wesentlich niedrigere spezifische Schadstoffemissionen ($NO_x$, HCl).

Im Falle der Verbrennung kann die Rückgewinnung des Lösungsmittels auch entfallen, um gegebenenfalls einen Verfahrensschritt einzusparen und gleichzeitig einen höheren Brennwert (Energieeinsparung) zu erreichen.

Die nachfolgenden Beispiele sollen die Erfindung näher erläutern.

## Beispiel 1

1 kg Produktgemisch, hergestellt nach dem in der DE-PS 33 46 910 beschriebenen Verfahren, bestehend aus 73,4 Gew.-% Guanidinhydrochlorid, 8,3 Gew.-% Thioharnstoff, 12 Gew.-% Silanen und 6,3 Gew.-% Ammoniumchlorid wird in 1 kg Aceton aufgeschlämmt und bei 25 °C 1 Stunde gerührt.

Der Feststoff wird abfiltriert (Filtrat I), erneut in 1 kg Aceton aufgeschlämmt, einige Minuten gerührt und das Endprodukt vom Filtrat (II) abgetrennt. Es wird mit 200 g frischem Aceton nachgewaschen.

Filtrat I enthält ca. 16 Gew.-% Feststoff. Nach Abziehen des Lösungsmittels verbleiben 190 g Feststoff mit 41 Gew.-% Thioharnstoff und 8 Gew.-% Guanidinhydrochlorid (Rest Silane).

Filtrat II enthält ca. 2 Gew.-% Feststoff.

Das Endprodukt (790 g) setzt sich zusammen aus ca. 91 Gew.-% Guanidinhydrochlorid, 8 Gew.-% Ammoniumchlorid, 0,3 Gew.-% Thioharnstoff und 0,5 Gew.-% sonstigen Verunreinigungen.

## Beispiel 2

1 kg Produktgemisch gleicher Zusammensetzung wie in Beispiel 1 werden in 1,1 kg Filtrat II aus Beispiel 1 aufgeschlämmt und bei 25 °C 1 Stunde gerührt. Der Feststoff wird abfiltriert, in 0,9 kg Aceton (zurückgewonnen aus Filtrat I in Beispiel 1) erneut aufgeschlämmt, einige Minuten gerührt und abfiltriert. Es wird mit 200 g frischem Aceton nachgewaschen.

Das Endprodukt (793 g) setzt sich zusammen aus ca. 91 % Guanidinhydrochlorid, 8 % Ammoniumchlorid, 0,4 % Thioharnstoff und 0,4 % sonstigen Verunreinigungen.

## Beispiel 3

300 kg Produktgemisch gleicher Zusammensetzung wie in Beispiel 1 werden in 300 kg Aceton aufgeschlämmt und bei Raumtemperatur 1 Std. gerührt. Der Feststoff wird abzentrifugiert. Es entstehen 224 kg kristallines, farbloses Produkt mit 0,8 % Aceton und 0,2 % Thioharnstoff. Der Feststoff wird ein weiteres Mal in 300 kg Aceton aufgeschlämmt, 1 Std. gerührt und abzentrifugiert, wobei 221 kg mit 1,3 % Aceton und 0,03 % Thioharnstoff anfallen.

**Ansprüche**

1. Verfahren zur Gewinnung von Guanidinhydrohalogeniden aus den bei der Herstellung von Mercaptoalkylsilanen anfallenden Nebenproduktgemischen bestehend im wesentlichen aus Guanidinhydrohalogenid, Ammoniumhalogenid, Thioharnstoff und Silanverbindungen,
**dadurch gekennzeichnet,**
daß man das Nebenproduktgemisch mit einem aliphatischen Keton mit bis zu sieben Kohlenstoffatomen extrahiert und den Extrakt von dem die Guanidinhydrohalogenide enthaltenden Rückstand abtrennt.

2. Verfahren nach Anspruch 1,
**dadurch gekennzeichnet,**
daß man Ketone verwendet, die drei bis vier Kohlenstoffatome und unverzweigte Reste aufweisen.

3. Verfahren nach Anspruch 1 oder 2,
**dadurch gekennzeichnet,**
daß man als Keton Aceton verwendet.

4. Verfahren nach einem der Ansprüche 1 bis 3,
**dadurch gekennzeichnet,**
daß man die Extraktion durch Digerieren mit der ein- bis zweifachen Menge an Keton, bezogen auf das Gewicht des Produktgemisches, durchführt.

5. Verfahren nach einem der Ansprüche 1 bis 4,
**dadurch gekennzeichnet,**
daß man die Extraktion bei Raumtemperatur durchführt.

6. Verfahren nach einem der Ansprüche 1 bis 5,
**dadurch gekennzeichnet,**
daß man die Extraktion in mehreren Stufen durchführt.

7. Verfahren nach den Ansprüchen 1 bis 3, 5 und 6,
**dadurch gekennzeichnet,**
daß man die Extraktion im Gegenstrom durchführt.

8. Verfahren nach den Ansprüchen 1 bis 7,
**dadurch gekennzeichnet,**
daß man das Keton nach der Extraktion zurückgewinnt.